# EUROPÄISCHE PATENTANMELDUNG

(11) **EP 2 980 069 A1**
(43) Veröffentlichungstag der Anmeldung: **03.02.2016**
(21) Anmeldenummer: 14179454.5
(22) Anmeldetag: 01.08.2014
(51) Int. Cl.: C07D 201/04, C07C 45/58, C07C 49/413

(54) **Verfahren zur Herstellung von Cyclododecanon**

(71) Anmelder: Evonik Degussa GmbH, 45128 Essen (DE)
(72) Erfinder: Micoine, Kévin, Dr., 45739 Oer-Erkenschwick (DE); Meier, Ralf, 44265 Dortmund (DE); Herwig, Jürgen, Dr., 46569 Hünxe (DE); Roos, Martin, Dr., 45721 Haltern am See (DE); Häger, Harald, Dr., 59348 Lüdinghausen (DE); Cameretti, Luca, Dr., 44267 Dortmund (DE); Döring, Jens, Dr., 44141 Dortmund (DE)

(57) **Zusammenfassung**

Gegenstand der Erfindung ist ein Verfahren zur Herstellung von Cyclodocecanon (CDON) umfassend die Schritte
a. Epoxidierung von Cyclododecen (CDEN) zu Epoxycyclododecan (CDANepoxid) und
b. Umlagerung des CDANepoxids zu CDON unter Erhalt eines Gemisches, das CDEN enthält.

Das CDEN wird aus dem CDON-haltigen Gemisch abgetrennt und der Epoxidierung zu CDANepoxid (Schritt a) zugeführt.

## Beschreibung

Die vorliegende Erfindung betrifft ein Verfahren zur Herstellung vor Cyclododecanon und ein Verfahren zur Herstellung von Laurinlactam.

Cyclododecanon (CDON) wird für die Synthese von Laurinlactam eingesetzt. Das Lactam wiederum eignet sich für die Herstellung von Polyamid 12.

Die Herstellung von CDON kann ausgehend von Cyclododecatrien (CDT) erfolgen. Zunächst kann eine Selektivhydrierung von Cyclododecatrien (CDT) in Cyclododecen (CDEN) vorgenommen werden. Anschließend folgen eine Epoxidierung von CDEN zu Epoxycyclododecan (CDANepoxid) und die Umlagerung von CDANepoxid zu Cyclododecanon (CDON). Ausgehend von CDEN umfasst die CDON-Synthese folgende Schritte:
a. Epoxidierung von Cyclododecen (CDEN) zu Epoxycyclododecan (CDANepoxid) und
b. Umlagerung des CDANepoxids zu CDON unter Erhalt eines Gemisches, das CDEN enthält.

Das Gemisch (CDON-haltiges Gemisch), das aus der Umlagerung erhalten wird, umfasst somit zumindest CDON und CDEN.

Bei diesem Herstellungsverfahren von CDON tritt das Problem auf, dass signifikante Mengen an CDEN entstehen können. Dieses Problem wird mit der Alterung von Katalysatoren, die gegebenenfalls eingesetzt werden, verschärft. Die anfallende Menge an CDEN kann mehr als 5 Gew.-%, bezogen auf das resultierende, CDON-haltige Gemisch aus der Umlagerung, betragen.

Der hohe Anteil an CDEN kann sich für die nachfolgenden Reaktionen wie die Herstellung von Laurinlactam nachteilig auswirken. Zudem sind geringe Produktausbeuten wenig wirtschaftlich.

Insofern bestand die Aufgabe der vorliegenden Erfindung darin, ein neues Verfahren zur Herstellung von CDON zur Verfügung zu stellen, durch das der Anteil an CDEN im Endprodukt CDON verringert und die Ausbeute an CDON erhöht wird. Zudem sollte das Herstellungsverfahren insgesamt wirtschaftlicher erscheinen.

Demgemäß wurde ein neues Verfahren zur Herstellung von CDON umfassend die eingangs genannten Schritte a und b gefunden, welches im Folgenden als Reaktionsroute I bezeichnet wird. Das CDEN wird aus dem CDON-haltigen Gemisch abgetrennt und der Epoxidierung zu CDANepoxid (Schritt a) zugeführt.

Das erfindungsgemäße Verfahren kann kontinuierlich oder diskontinuierlich durchgeführt werden.

Vorzugsweise wird das CDEN für die Epoxidierung (Schritt a) aus CDT erhalten. Das CDT wiederum kann aus 1,3-Butadien gewonnen werden. Die Selektivhydrierung von CDT kann in der Gasphase mit einem niedrigen Partialdruck an Wasserstoff und einem Pd-haltigen Katalysator erfolgen (EP 1457476). Aus dem Schritt entstehen üblicherweise 5 bis 15 Gew.-% CDAN (Cyclododecan) in CDEN.

Die Epoxidierung von CDEN gemäß Schritt a kann mit Wasserstoffperoxid, mittels eines Phasentransferkatalysators und eines Metallsalzes im sauren pH durchgeführt werden. Das im CDEN enthaltende CDAN wirkt als Absetzbeschleuniger (EP 1411050, EP 1411051). Nach der Reaktion wird CDANepoxid erhalten, das weiterhin CDAN, unreagiertes CDEN oder beides umfassen kann.

CDEN, dass nicht zu CDANepoxid umgesetzt worden ist (unreagiertes CDEN), kann vor der Umlagerung (Schritt b) zumindest teilweise abgetrennt werden.

Das nach der Epoxidierung vorliegende CDAN ist zumindest teilweise abzutrennen, damit CDAN durch den Kreislauf nicht aufkonzentriert wird. Vorzugsweise wird mindestens 50 Gew.-% des CDAN, bezogen auf das Gesamtgewicht an CDAN, bevorzugt 90 Gew.-%, besonders bevorzugt 95 Gew.-%, ganz besonders bevorzugt 98 Gew.-% und insbesondere 100 Gew.-% abgetrennt werden. Der abzutrennende Anteil an CDAN kann insbesondere in den Fällen, in denen kein oder wenig CDEN vorhanden ist, mindestens 90 Gew.-% betragen. Wenig CDEN ist vorhanden, wenn der Anteil an CDEN in der Mischung aus CDANepoxid, CDAN und CDEN weniger als 5 Gew.-% beträgt.

Sofern unreagiertes CDEN nach der Epoxidierung vorliegt, kann CDEN als Gemisch mit CDAN zumindest teilweise wieder der Epoxidierung oder ohne Abtrennung der Umlagerung (Schritt b) zugeführt werden. Abgetrenntes CDAN kann anschließend zu CDON oxidiert werden, wobei zumindest teilweise CDOL entstehen kann. Das CDOL kann danach zu CDON dehydriert werden.

Die Umlagerung gemäß Schritt b erfolgt vorzugsweise in Gegenwart eines edelmetallhaltigen Katalysators, wobei der Katalysator bevorzugt Titandioxid, Zirconiumdioxid oder beides enthält. In diesem Reaktionsschritt bildet sich CDON, das weiterhin CDAN, CDEN, CDOL oder Mischungen daraus als Nebenprodukte enthalten kann (CDON-haltiges Gemisch). Ebenso können weitere Nebenprodukte enthalten sein, die einen gegenüber CDON höheren Siedepunkt aufweisen (Hochsieder).

Das CDEN wird abgetrennt und der Epoxidierung zugeführt. Sofern CDAN enthalten ist, wird dieses der Epoxidierung üblicherweise als Gemisch mit CDEN zugeführt.

Das verbleibende Gemisch umfassend CDON und Hochsieder einschließlich CDOL kann in Gegenwart von Wasserstoff und einem Katalysator hydriert werden, um die ungesättigten Nebenprodukte zu entfernen. Anschließend wird das reine Produkt CDON von einer Hochsiederfraktion einschließlich CDOL beispielsweise per Destillation abgetrennt.

Aus dieser Hochsiederfraktion kann CDOL anschließend abdestilliert werden und CDOL kann innerhalb der Reaktionsroute I mittels eines Dehydrierungskatalysators in CDON umgesetzt werden. Es ist jedoch bevorzugt, das abgetrennte CDOL vor der Dehydrierung einer Reaktionsroute II zur Herstellung von CDON zuzuführen.

Die Reaktionsroute II umfasst die folgenden Schritte:
a. Hydrierung von CDT zu CDAN,
b. Oxidation von CDAN zu einem Gemisch umfassend CDOL und CDON und
c. Dehydrierung von CDOL zu CDON.

Geeignete Katalysatoren für die Dehydrierung von CDOL enthalten Kupfer oder Kupferverbindungen wie beispielsweise Kupfer-(II)-oxid.

Das CDOL aus der Route I wird vorzugsweise vor Schritt c der Route II, der Dehydrierung, eingespeist.

Ebenso kann das aus der Route I abgetrennte CDAN vor Durchführung der Oxidation der Reaktionsroute II zugeführt werden. Hierbei wird das CDAN vorzugsweise vor dem Schritt b der Route II, der Oxidation, zugeführt. Das CDAN kann beispielsweise nach der Epoxidierung der Route I abgetrennt werden.

Insofern können die Reaktionsrouten I und II in der Art kombiniert werden, dass anfallendes CDAN bzw. CDOL aus der Route I abgetrennt und entfernt und in Route II überführt werden. Hierbei ist es bevorzugt, die beiden Routen kontinuierlich durchzuführen. Diese besondere Ausführungsform der Erfindung nutzt Nebenprodukte der Route I zur Weiterverarbeitung in Route II. Dies ist besonders wirtschaftlich und ökologisch. Eine Entsorgung der Nebenprodukte der Route I entfällt.

Die Abtrennung von CDEN, CDAN bzw. CDOL sowie der weiteren Hochsieder kann durch dem Fachmann geläufige Methoden vorgenommen werden. Hierbei ist die Destillation bevorzugt. Besonders bevorzugt erfolgen sämtliche Abtrennungen mittels Destillation. Es ist vorteilhaft, mehrere Destillationen nacheinander durchzuführen (mehrstufige Destillation).

Das CDAN kann nach der Epoxidierung abdestilliert werden.

Nach der Umlagerung ist es vorteilhaft, zunächst ein Gemisch aus CDAN und CDEN (Leichtsiederfraktion) abzudestillieren und den Rückstand enthaltend CDON, CDOL und weitere Hochsieder erneut einer Destillation zu unterziehen. Hierbei lässt sich CDON gewinnen, das von einer Hochsiederfraktion enthaltend CDOL abgetrennt wird. CDOL wiederum kann von den verbleibenden Hochsieder-Produkten abdestilliert werden.

Leichtsieder sind Stoffe mit einem Siedepunkt, der geringer ist als der Siedepunkt von CDON.

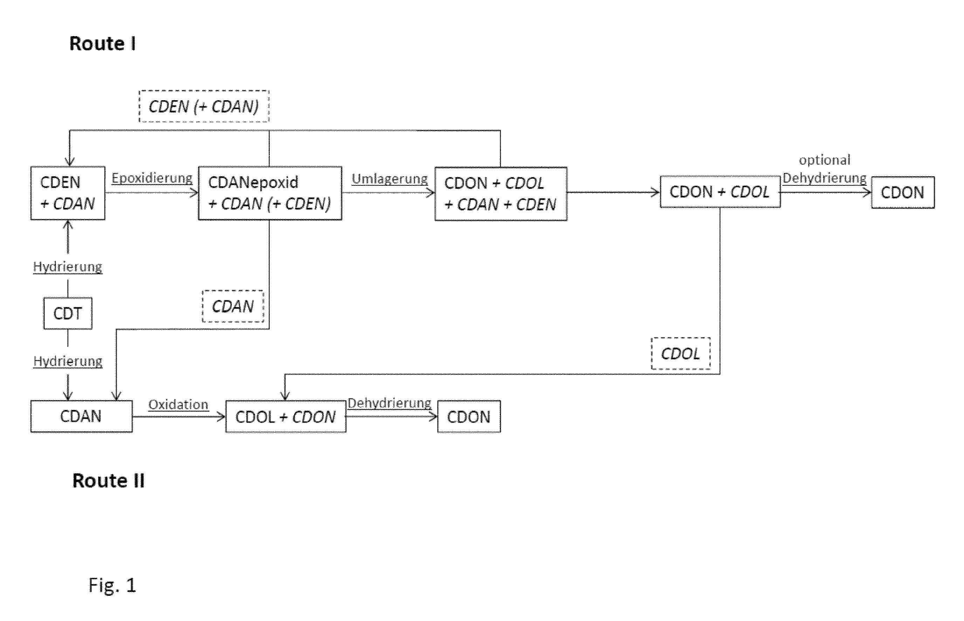

Die Fig. 1 stellt den Stoffverlauf mit den entsprechenden Reaktionen dar. Die kursiv darstellten Verbindungen sind Nebenprodukte der jeweiligen Reaktion.

Ausgehend von CDT wird via Route I mittels Hydrierung (Selektivhydrierung) CDEN erhalten, das zu CDANepoxid epoxidiert wird. Anschließend erfolgt eine Umlagerung zu CDON, wobei vorhandenes CDEN - beispielsweise mittels Destillation - abgetrennt und dem CDEN vor der Epoxidierung zugeführt wird. Das nach der Epoxidierung enthaltende CDEN kann ebenfalls zurückgeführt werden. Das zurückgeführte CDEN kann CDAN enthalten.

Das verbleibende CDON kann CDOL enthalten, dass abgetrennt und der Route II zugeführt werden kann. Alternativ kann das CDOL innerhalb der Route I zu CDON dehydriert werden.

Ein weiterer Gegenstand der Erfindung ist ein Verfahren zur Synthese von Laurinlactam (erfindungsgemäßes Lactamverfahren), bei dem das zuvor genannte erfindungsgemäße Verfahren zur Herstellung von CDON herangezogen wird.

Das im erfindungsgemäßen Verfahren produzierte CDON kann im erfindungsgemäßen Lactamverfahren unter Erhalt von Cyclododecan-oxim (CDON-oxim) oximiert werden. Im Folgeschritt kann die Beckmann-Umlagerung zum Laurinlactam erfolgen, wobei die Umlagerung mittels Schwefelsäure oder Cyanurchlorid erfolgen kann. Das Lactam kann unter Polykondensation zu Polyamid weiterverarbeitet werden.

Die Oximierung, die Beckmann-Umlagerung sowie die Kondensationsreaktion sind dem Fachmann bekannt.

Auch ohne weitere Ausführungen wird davon ausgegangen, dass ein Fachmann die obige Beschreibung im weitesten Umfang nutzen kann. Die bevorzugten Ausführungsformen und Beispiele sind deswegen lediglich als beschreibende, keinesfalls als in irgendeiner Weise limitierende Offenbarung aufzufassen.

Nachfolgend wird die vorliegende Erfindung anhand von einem Beispiel näher erläutert. Alternative Ausführungsformen der vorliegenden Erfindung sind in analoger Weise erhältlich

### Beispiele

Sofern nichts anderes angegeben ist, beziehen sich die prozentualen Angaben auf Massen

### Selektivhydrierung von CDT:

1000 kg/h CDT wurden in einer Sättigungskolonne als Gas-Flüssigkontaktapparat bei einem Druck von 0,75 bar und einer Temperatur von 155°C kontinuierlich in einen 10 m³/h Kreisgasstrom, der im Wesentlichen aus Stickstoff bestand, verdampft. Der sich ergebende Gasstrom wurde einem Festbettreaktor mit einem Pd auf Al₂O₃ Katalysator zugeführt, in dem bei einer Temperatur von 130 °C die Reaktion mit Wasserstoff erfolgte. Dazu wurden 25 kg/h Wasserstoff am Eingang des Reaktors zum Kreisgasstrom dosiert. Am Ausgang des Reaktors wurde aus dem Kreisgasstrom das Produkt bei 35 °C auskondensiert. Die auskondensierte Flüssigkeit hatte eine Zusammensetzung von 84,8 % CDEN und 15 % CDAN. CDDIEN- und CDT-Isomere liegen in Konzentrationen kleiner 0,2 % vor.

Der verbleibende Kreisgasstrom wurde über einen Verdichter wiederum der Sättigungskolonne zugeführt (siehe oben).

### Epoxidierung von CDEN:

Die Mischung von CDEN (84,8 %) und CDAN (15 %) aus der Selektivhydrierung wurde mit einer Dosierrate von 2 kg/h in einer dreistufigen Reaktorkaskade mit H₂O₂ epoxidiert. In die Reaktorkaskade wird zusätzlich eine Mischung von CDEN (76 %) und CDAN (24 %) aus der Leichtsiederdestillation (Destillation 1) des Umlagerungsschritts mit einer Dosierrate von 80 g/h zurückgeführt.

Dieses CDAN/CDEN Gemisch wurde zuerst in einen ersten 8 L-Reaktor kontinuierlich mit einer Dosierrate von 2,08 kg/h zudosiert. Es wurde zusätzlich eine 50 %ige H₂O₂ Lösung (560 g/h), eine wässrige Lösung von 30 Gew.-% Na₂WO₄ und 18 Gew.-% H₃PO₄ (130 g/h), eine 50 %ige Lösung von Trioctylamin in CDEN (70 g/h) und Wasser (150 g/h) in das 5,5 L zweiphasige Reaktionsgemisch bei einer Temperatur von 80 °C zudosiert. Die zweiphasige Mischung kam aus dem ersten Reaktor mit einem Umsatz des CDEN von 79 % und lief in einen zweiten 8 L-Reaktor über. Dazu wurde eine 50 %ige H₂O₂ Lösung (135 g/h) in das 5,5 L zweiphasige Reaktionsgemisch bei einer Temperatur von 78 °C zudosiert.

Aus diesem zweiten Reaktor kam eine zweiphasige Mischung mit einem Umsatz des CDEN von 96% und wurde in einen Reaktor mit einem Füllstand von 25 L geleitet. Das zweiphasige Reaktionsgemisch wurde in diesem Reaktor bei einer Temperatur von 76 °C gerührt.

Die beiden Phasen der Reaktionsmischung aus dem 25 L-Reaktor wurden in einem Phasentrennbehälter getrennt. Die organische Phase wurde anschließend mit einer 5 %ige NaOH Lösung (0,5 kg/h) in einem 5 L-Behälter gewaschen und beide Phasen wurden in einem Trennbehälter getrennt.

Die organische Phase wurde letztendlich in drei Stufen kontinuierlich destilliert. In einer ersten Kolonne (Kolonne mit 8 m Gewebepackung; Oberfläche von 500 m²/m³; Kopfdruck 300 mbar) wurde das restliche Wasser ins Destillat abgetrennt. In einer zweiten Kolonne (Kolonne mit 6 m Gewebepackung; Oberfläche von 500 m²/m³; Kopfdruck 10 mbar) wurde das CDAN (320 g/h) abdestilliert und das CDAN-Epoxid im Sumpf gesammelt. In einer dritten Kolonne (Kolonne mit 6 m Gewebepackung; Oberfläche von 500 m2/m3; Kopfdruck 10 mbar) wurde das gewünschte Produkt CDAN-Epoxid (Reinheit > 99,5 %) mit einer Rate von 1,73 kg/h im Destillat erhalten. Es entspricht eine gesamte Ausbeute von 98 % für die Epoxidierung.

### Umlagerung von CDAN-Epoxid in CDON:

Das CDAN-Epoxid aus dem vorherigen Schritt wurde mittels eines 0,5 % Pd/ZrO₂ Katalysators in einer dreistufigen Reaktorkaskade in CDON umgesetzt.

Das CDAN-Epoxid aus der Epoxidierung wurde in einen ersten Kreislaufreaktor mit einer Dosierrate von 1,73 kg/h zudosiert. Der Kreislaufreaktor bestand aus einem 12 L-Rohreaktor, der mit 10 kg 0,5 % Pd/ZrO₂ Festbettkatalysator bei einer Temperatur von 220 °C gefüllt war und aus einem 8 L-Vorlagebehälter gespeist wurde.

Aus dem ersten Kreislaufreaktor kam eine Mischung von 65 % CDON, 21 % CDAN-Epoxid, 0,9 % CDAN, 2,8 % CDEN, 2,5 % CDOL und 7,8 % Neben- und Zwischenprodukte. Dieses Gemisch wurde in einen zweiten Kreislaufreaktor mit einer Dosierrate von 1,73 kg/h zudosiert. Dieser zweite Kreislaufreaktor bestand ebenfalls aus einem 8 L-Vorlagebehälter und einem 12 L-Rohreaktor, der mit 10 kg 0,5 % Pd/ZrO₂ Festbettkatalysator bei einer Temperatur von 220 °C gefüllt war.

Aus dem zweiten Reaktionskreislauf kam eine Mischung enthaltend 88 % CDON, 4 % CDAN-Epoxid, 1,1 % CDAN, 3,4 % CDEN und 2,9 % CDOL. Dieses Gemisch wurde in ein mit 2,7 kg Pd/ZrO₂ gefülltem Festbettreaktor mit einer Dosierrate von 1,73 kg/h zudosiert. In diesem Rohr wurde die Reaktionsmischung bei einer Temperatur von 230 °C aufgeheizt. Aus diesem Rohr wurde eine Rohmischung mit 92 % CDON, 1,1 % CDAN, 3,5 % CDEN und 2,2 % CDOL erhalten. Die Komponenten dieses Gemisches wurden mittels Destillation voneinander getrennt und aufgereinigt.

### Destillation Leichtsiederfraktion:

Die Rohmischung aus der Umlagerung wurde in einem ersten kontinuierlichen Destillationschritt von einer Leichtsiederfraktion (Komponenten, die einen geringeren Siedepunkt als CDON besitzen) befreit. Die verwendete Kolonne war mit 8 m Gewebepackung mit einer Oberfläche von 500 m²/m³ ausgerüstet und wurde bei einem Kopfdruck von 10 mbar betrieben. Das Destillat (80 g/h) mit 76% CDEN und 24% CDAN wurde in die Epoxidierung von CDEN zurückgeführt (siehe oben). Der Sumpf wurde in die zweite Destillationskolonne zugeführt.

### Destillation von CDON:

Der Sumpf aus der ersten Destillation wurde in einem weiteren kontinuierlichen Destillationschritt (Kolonne mit 6 m Gewebepackung; Oberfläche von 500 m²/m³) destilliert und CDON bei einem Kopfdruck von 10 mbar als Destillat gewonnen. Komponenten mit einem höheren Siedepunkt als CDON (zum Beispiel CDOL) wurden als Sumpfprodukt abgetrennt. Die Reinheit des gewonnen CDON betrug > 99% und als CDON-Ausbeute wurden in der Destillation 98% erreicht.

Dieses Cyclododecanon konnte mit bekannten Verfahren weiter zu Laurinlactam umgesetzt werden.

Der Sumpf dieser zweiten Destillation wurde in einer zusätzlichen Kolonne weiter aufgereinigt: CDOL wurde in einer zusätzlichen Kolonne von anderen Hochsiedern abgetrennt und konnte mittels Dehydrierung zu CDON umgesetzt werden.

## Patentansprüche

1. Verfahren zur Herstellung von Cyclodocecanon (CDON) mittels einer Reaktionsroute I, umfassend die Schritte
a. Epoxidierung von Cyclododecen (CDEN) zu Epoxycyclododecan (CDANepoxid) und
b. Umlagerung des CDANepoxids zu CDON unter Erhalt eines Gemisches, das CDEN enthält,
**dadurch gekennzeichnet, dass** CDEN aus dem CDON-haltigen Gemisch abgetrennt und der Epoxidierung zu CDANepoxid (Schritt a) zugeführt wird.

2. Verfahren nach Anspruch 1, **dadurch gekennzeichnet, dass** CDEN für die Epoxidierung (Schritt a) aus Cyclododecatrien (CDT) erhalten wird.

3. Verfahren nach einem der vorherigen Ansprüche, **dadurch gekennzeichnet, dass** die Umlagerung (Schritt b) in Gegenwart eines edelmetallhaltigen Katalysators erfolgt.

4. Verfahren nach Anspruch 3, **dadurch gekennzeichnet, dass** der Katalysator der Umlagerung (Schritt b) Titandioxid, Zirconiumdioxid oder beides enthält.

5. Verfahren nach einem der vorherigen Ansprüche, **dadurch gekennzeichnet, dass** das CDANepoxid aus dem Schritt a CDEN enthält, das vor der Umlagerung (Schritt b) zumindest teilweise abgetrennt wird.

6. Verfahren nach einem der vorherigen Ansprüche, **dadurch gekennzeichnet, dass** das CDANepoxid aus dem Schritt a Cyclododecan (CDAN) enthält, das vor der Umlagerung (Schritt b) zumindest teilweise abgetrennt wird.

7. Verfahren nach Anspruch 6, **dadurch gekennzeichnet, dass** CDAN zu CDON oxidiert wird.

8. Verfahren nach einem der vorherigen Ansprüche, **dadurch gekennzeichnet, dass** das CDON-haltige Gemisch Cyclododecanol (CDOL) enthält, das zu CDON dehydriert wird.

9. Verfahren nach Anspruch 8, **dadurch gekennzeichnet, dass** das CDOL vor der Dehydrierung aus dem CDON-haltigen Gemisch abgetrennt und einer Reaktionsroute II zur Herstellung von CDON zugeführt wird, welche umfasst
a. Hydrierung von CDT zu CDAN,
b. Oxidation von CDAN zu einem Gemisch umfassend CDOL und CDON und
c. Dehydrierung von CDOL zu CDON.

10. Verfahren nach einem der Ansprüche 6 bis 10, **dadurch gekennzeichnet, dass** CDAN einer Reaktionsroute II zur Herstellung von CDON zugeführt wird, welche umfasst
a. Hydrierung von CDT zu CDAN,
b. Oxidation von CDAN zu einem Gemisch umfassend CDOL und CDON und
c. Dehydrierung von CDOL zu CDON.

11. Verfahren nach einem der vorherigen Ansprüche, **dadurch gekennzeichnet, dass** mindestens eine Abtrennung, vorzugsweise alle Abtrennungen, mittels Destillation erfolgen.

12. Verfahren nach einem der vorherigen Ansprüche, **dadurch gekennzeichnet, dass** CDT aus 1,3-Butadien erhalten wird.

13. Verfahren zur Synthese von Laurinlactam, bei dem ein Verfahren zur Herstellung von CDON gemäß einem der vorherigen Ansprüche herangezogen wird.

14. Verfahren nach Anspruch 13, **dadurch gekennzeichnet, dass** das CDON zu Cyclododecanon-oxim (CDON-oxim) umgesetzt wird.
